Europäisches Patentamt

European Patent Office

Office européen des brevets

(19)

(11) Publication number: **0 362 408**
**A1**

(12)

# EUROPEAN PATENT APPLICATION
## published in accordance with Art. 158(3) EPC

(21) Application number: **89903796.4**

(22) Date of filing: **23.03.89**

(86) International application number:
**PCT/JP89/00306**

(87) International publication number:
**WO 89/08977 (05.10.89 89/24)**

(51) Int. Cl.5: **A01G 1/00 , C12M 3/00**

(30) Priority: **25.03.88 JP 69844/88**

(43) Date of publication of application:
**11.04.90 Bulletin 90/15**

(84) Designated Contracting States:
**DE FR GB IT**

(71) Applicant: **P.C.C. TECHNOLOGY INC.**
**8-7, Shibuya 2-chome**
**Shibuya-ku, Tokyo 150(JP)**

(72) Inventor: **TAKAYAMA, Shinsaku**
**2-11-2, Matsushiro**
**Tsukuba-shi Ibaraki 305(JP)**

(74) Representative: **Myerscough, Philip Boyd et al**
**J.A.Kemp & Co. 14, South Square Gray's Inn**
**London, WC1R 5EU(GB)**

(54) **METHOD OF CULTURING PLANT ORGANS AND CULTURE VESSEL THEREFOR.**

(57) This invention relates to a method of culturing plant organs and a culture device used therefor. According to the present invention, a culture vessel in which both (I) the agitating blades (2) extending from a rotary shaft (1) provided in the central portion of the culture vessel, and (II) the fixed baffle terminals (4) extending inward from positions in the vicinity of the outer wall of the culture vessel are provided in a comb-like arrangement is used so as to prevent the formation of agglomerate of plant organs, which causes a decrease in the plant organ culturing efficiency. The culturing of plant organs is done as the agglomerate is disunited by the rotation of the agitating blades (2). The method and device according to this invention are also used for culturing a large quantity of microorganisms besides plant organs.

TITLE

SPECIFICATION

# METHOD FOR CULTURING PLANT ORGAN AND CULTURE TANK THEREFOR

## Technical Field of the Invention

The present invention relates to a method for culturing plant organs in large quantities and a culture tank for the method.

## Background of the Invention

With regard to the culture tanks used for cultivating plant tissue, in addition to the culture tanks of aerated agitation type and air-lift type that are presently utilized, such culture equipment as gas-phase culture equipment (Japanese Laid-Open Patent Publications Nos. Sho 59(1984)-45873, Sho 59(1984)-45879), rotary drum type culture tank (H. Tanaka et al., Biotechnology and Bioengineering, Vol. 25, pp.2359, 1983), spin filter type culture tank (D.J. Styer et al., Tissue Culture in Forestry and Agriculture, pp.117, 1985 published by Plenum & Press), etc. are already known and have been utilized mainly as the methods for culturing plant cells. Plant cells usually form aggregate of several mm or smaller in size with rare exception of about 2 to 3 cm at the largest, whereas plant organs such as root, stem, leaf, plant body, etc. are sometimes cultured into much larger aggregates than those of plant cells with ordinary size of several cm or larger and

- 1 -

further size of several ten cm as the case may be. In case
that the conventional culture tanks are used for cultivating
plant organs, therefore, the cultured plant organs form
aggregates, causing a marked inhibition of growth because of
strong shearing stress acted upon the plant organs even if
aerated agitation is carried out. Accordingly, it is not
easy to effectively culture plant organs so long as the
conventional culture equipment is used. Among the culture
equipment, only the above-mentioned gas-phase culture
equipment, rotary drum type culture tank and spin filter
type culture tank seem to be somewhat useful. However,
since a culture tank is generally quite expensive in
construction cost, it is desirable that the culture tank can
be effectively used for culturing not only plant organs but
also plant cells and, if required, microorganism to avoid
the ineffective construction of a culture tank for the sole
purpose of culturing plant organs. With respect to the
conventional tanks, a tank which is capable of being
generally utilized for culturing microorganism, plant cells
and plant organs has not yet been developed. The
conventional culture tanks, when used for culturing plant
organs, frequently cause aggregates of several cm to several
ten cm on larger in diameter with the well-grown plant
organs, which lead to death because of lack of oxygen. The
above-mentioned large aggregates are serious obstacles in mass culture of plant organs. Furthermore, nothing is known regarding the conventional culture tank capable of effectively culturing plant organs without causing mechanical damage to the organs and forming aggregation of such organs.

Disclosure of the Invention

The present invention relates to a method for culturing plant organs characterized in that aggregates of plant organs are disassembled completely with an agitator during culturing and to a culture tank equipped with an agitator capable of disassembling the aggregates of plant organs. More particularly, the present invention pertains to a culture tank equipped with an agitator characterized in that an agitating impeller and a baffle terminal are alternately installed on the agitator, and also to a culture tank for culturing plant organs characterized in that the clearance between the agitating impeller and the baffle terminal coincides with the size of each plant organ.

Brief Description of the Drawing ---

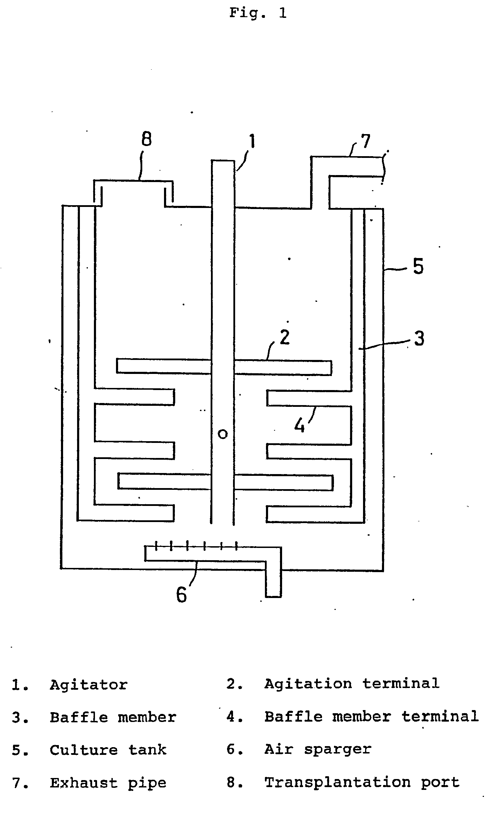

Fig. 1 illustrates an example of disassembling apparatus for plant organs used in the present invention. Part number:

1 ... Agitator, 2 ... Terminal, 3 ... Baffle member

4 ... Terminal, 5 ... Culture tank, 6 ... Air sparger

7 ... Exhaust pipe, 8 ... Transplantation port

Best Mode of Carrying Out the Invention ---

A culture tank of the present invention is exemplified in Fig. 1. An agitator 1 is equipped with agitation terminals 2, each being in the form of bar, plate, long and slender member, or else. The number of the agitation terminals may be arbitrarily selected, but can be experimentally obtained according to the size of the tank as well as the degree of disassembling of plant organs to be attained.

In order to improve the effect of disassembling, it is preferable that baffle member/s be installed inside a culture tank, for instance, a baffle member 3 is installed as shown in Fig. 1.

The agitator and baffle member are positioned so as not to come in contact with each other. Thus, the minimum distance in the space between the agitation terminal and the baffle member terminal 4 coincides with the approximate size of each disassembled plant organ.

In addition to the above members and parts, the culture tank is equipped with an air sparger 6, exhaust pipe 7, transplantation port 8, etc.

In case of culturing by use of the above-mentioned equipment, it is preferable that the agitator be operated at a revolution in the range of 10 to 60 rpm for 20 minutes to 5 hours with a period of 1 to 5 days.

- 4 -

The agitator may be installed in any position in the tank and preferably is of such structure as being kept off from culture liquid except during operation. Those skilled in the art can easily design improved culture tanks over the above ones.

The culture tank of the present invention can be generally used as a culture tank not only for culturing plant organs but also for mass culture microorganism. The use of the equipment of the present invention for the purpose of culturing enables efficient and mass culture of plant organs without causing aggregates of and damage to plant organs.

The plant organs that are used in the present invention are exemplified by the organs of such plants as classified into pteridophyte, gymnosperm or angiosperm, and usually by leaf, stem, bud, growing point, root, bulb, embryo, etc. of such plants. The foregoing plant organs are substantially capable of being cultured massively, but the preferable organs are those having a property of forming large aggregate. Plant organs, after being cultured in solid or liquid form for proliferation, are cultured in the culture equipment of the present invention equipped inside with disassembling apparatus for plant organs. It goes without saying that after being cultured in the culture equipment of the present invention for proliferation, the plant organs

- 5 -

can be transplanted to another culture equipment of the present invention for further repeated culturing. In this case, culturing is carried out, for instance, by the following manner:

Any culture medium to be used for culturing and proliferating plant organs can be utilized, provided that the medium possesses a composition basically suitable for culturing plant tissues. In more details, a culture medium containing a moderate amount of sugar in the range of 10 to 100 g/ℓ, plant hormones in the range of 0.1 to 10 mℓ, nitrogen source, inorganic substance, vitamins, etc. can be used, regardless of the origin whether natural medium or synthetic one.

Examples of sugars are sucrose, glucose, lactose, maltose, etc. Plant hormones can be, for example, auxins such as α-naphthaleneacetic acid, 2,4-dichlorophenoxyacetic acid, indoleacetic acid, indolebutyric acid etc., cytokinins such as kinetin, benzyladenine, zeatin, 4 PU, etc., gibberellins such as GA3, GA4, Ga7, etc., abscisic acid, ethylene, etc. Examples of nitrogen sources include potassium nitrate, sodium nitrate, ammonium nitrate, calcium nitrate, ammonium sulfate, amino acids such as glycin, glutamic acid, lysine, aspartic acid, etc., yeast extract, meat extract, peptone, etc.

Inorganic substances may be, for example, potassium

- 6 -

chloride, calcium chloride, manganese chloride, nickle chloride, cobalt chloride, aluminium chloride, iron chloride, magnesium sulfate, sodium sulfate, nickle sulfate, iron sulfate, manganese sulfate, titanium sulfate, zinc sulfate, copper sulfate, sodium dihydrogen phosphate, potassium dihydrogen phosphate, potassium iodide, boric acid, sodium molybdate, etc.

Other substances such as vitamin B, inositol, pyridoxine hydrochloride, nicotinic acid, thiamine hydrochloride, biotin, etc. may be added to culture medium according to the demand.

Examples of culture media to be used are culture medium of Murashige Skoog, that of Linsmaier - Skoog, that of White that of Knopp, etc.

Most of culture is carried out at a temperature of 10 to 35°C, an illuminance of 0 to 20,000 lux, and a pH of 3.5 to 8.5 during a culture period of 10 to 100 days.

The general method of culturing plant organs is described as follows along with the method of the present invention wherein plant organs are cultured with disassembling:

Basically a known method is being used for culturing plant organs. For example, plant organs are prepared, proliferated and cultured with the procedures as described hereunder:

Firstly, plant tissues such as leaf, stem, root, etc. are cut into small pieces (5x5 to 50x50 mm), the surfaces of the pieces are sterilized with sodium hypochlorite, ethyl alcohol or the like, and then thoroughly washed with sterilized water. The surface-sterilized small pieces are placed in a sterilized culture medium in solid state at a density of one piece per 2 to 10 m$\ell$ of culture medium, and allowed to stand for 20 to 50 days at 10 to 35°C for culturing. Then, aggregates of differentiated tissues of stem, leaf, root, etc. are obtained. The aggregates are transplanted for liquid culturing to a flask or a culture tank containing sterilized liquid culture medium to be used for plant-tissue culture. The culturing in liquid culture medium is conducted, for example, in a 300 m$\ell$ erlenmeyer flask by means of transplanting thereto about 30 to 200 m$\ell$ of liquid culture medium and 1 to 5 numbers of the above tissue aggregates per 100 m$\ell$ of the culture medium and culturing at 10 to 35°C with shaking at 60 to 250 rpm. In case that a culture tank, for example, a 3 m$\ell$ culture tank is used, 1 to 2 $\ell$ of culture medium and 1 to 5 numbers of the above aggregates of the differentiated tissues per 100 m$\ell$ of the culture medium are placed in a culture tank, and cultured at 10 to 35°C with aeration at a rate of 0.5 to 3 $\ell$ per minute of sterilized air. When the transplanted and differentiated tissues have grown as large as 2 to 20 times the transplanted amount by virtue of the liquid culturing in the flask or culture tank, the grown differentiated tissues are divided into 2 to 20 pieces, and transplanted to the liquid culture medium in a flask or a culture tank in the same manner as above at a density of 1 to 5 numbers of the divided tissues per 100 mℓ of the liquid culture medium, followed by repeating the culturing procedure under the same condition as described hereinbefore to proliferate the differentiated tissues. Aside from the above methods, the known methods that are disclosed in Japanese Laid-Open Patent Publications Nos. Sho 54(1979)-40138, Sho 55(1980)-15734, Sho 55(1980)-118319, Sho 61(1986)-36022, etc. can be utilized without modification.

The cultured products obtained by the above culturing are transplanted and subjected to mass culture.

An example of disassembling apparatus for plant organs used in the present invention is illustrated in Fig. 1.

The examples which follow illustrate the invention in more detail.

Example 1 ---

A belladonna stem of about 5 cm in length is sterilized with 70% ethyl alcohol for 2 minutes and then with aqueous solution of sodium hypochlorite (available chlorine of 0.5%) for 100 minutes, and thereafter cut into sections of 5 to 10 mm. The sections are transplanted in a test tube of 24 mm in diameter and 125 mm in length containing 10 mℓ culture medium which has been prepared by adding to Murashige-Skoog culture medium as shown in Table-1, N-(2-chloro-4-pyridyl)N-phenylurea in 1 mg per ℓ of culture medium and agar-agar in 8 g per ℓ of culture medium, and then cultured at 22°C for 30 days under continuous illumination of 2500 lux.

After the above 30 days of culturing, the grown tissues are sterilely taken out, and only the roots generated from the tissues are sterilely collected with a pincette and a knife. These roots are again transplanted in a conical beaker containing 100 mℓ culture medium which is newly prepared according to the composition as shown in Table-2, and cultured at 22°C for 30 days to obtain masses of grown roots. The masses are sterilely divided with a pincette and a knife, and subjected to subculture repeatedly in the same manner as above by use of the culture medium as shown in Table-1 to proliferate the roots only. The roots thus proliferated are sterilely taken out, and only the roots generated from the tissues are sterilely collected with a pincette and a knife. The roots contained in 4 conical beakers are transplanted in a culture tank of the present invention with 10 parts in volume as shown in Fig. 1 which contains 8 parts in volume of the liquid culture medium having the same composition as that in Table-2 except that

- 10 -

60.0 g sucrose and 0.3 mg α-naphthaleneacetic acid are used, and cultured at 22°C for 40 days by means of operating the disassembling apparatus as shown in Fig. 1 at 30 rpm for 2 hours once per two days. As a result, the roots are subjected to branch proliferation and uniformly dispersed entirely in the culture tank without forming aggregates. The roots in the culture tank amount to 3700 g in gross weight (210 g as dry weight).

On the other hand, the use of a culture tank which is not equipped inside with disassembling apparatus results in floatage of the roots immediately below the liquid level, growth of the roots filling in the culture tank and death of the roots inside the aggregates. The roots thus grown and obtained in the culture tank amount to only 2400 g in gross weight (130 g as dry weight).

### Table-1  Modified Murashige-Skoog Culture Medium

(Unit: mg, unless otherwise noted)

| | |
|---|---|
| Ammonium nitrate | 825 |
| Potassium nitrate | 950 |
| Calcium chloride, dihydrate | 220 |
| Magnesium sulfate, heptahydrate | 185 |
| Potassium phosphate, monobasic | 85 |
| $Na_2 \cdot EDTA$, dihydrate | 18.65 |
| Ferrous sulfate, heptahydrate | 13.9 |
| Boric acid | 3.1 |
| Manganese sulfate, tetrahydrate | 11.15 |
| Zinc sulfate, heptahydrate | 4.3 |
| Potassium iodide | 0.415 |
| Sodium molybdate, dihydrate | 0.125 |
| Cuprous sulfate | 0.0125 |
| Cobalt chloride | 0.0125 |
| Vitamin B1 | 0.2 |
| Inositol | 50.0 |
| Pyridoxine hydrochloride | 0.25 |
| Nicotinic acid | 0.25 |
| Glycin | 1.00 |
| Sucrose | 30.0 g (gram) |
| Naphtaleneacetic acid | 0.1 |

- 12 -

## Table-2  Murashige-Skoog Culture Medium

(Unit: mg, unless otherwise noted)

| | |
|---|---|
| Ammonium nitrate | 1,650 |
| Potassium nitrate | 1,900 |
| Calcium chloride, dihydrate | 440 |
| Magnesium sulfate, heptahydrate | 370 |
| Potassium phosphate, monobasic | 170 |
| $Na_2 \cdot EDTA$, dihydrate | 37.3 |
| Ferrous sulfate, heptahydrate | 27.8 |
| Boric acid | 6.2 |
| Manganese sulfate, tetrahydrate | 22.3 |
| Zinc sulfate, heptahydrate | 8.6 |
| Potassium iodide | 0.83 |
| Sodium molybdate, dihydrate | 0.25 |
| Cuprous sulfate | 0.025 |
| Cobalt chloride | 0.025 |
| Vitamin B1 | 0.40 |
| Inositol | 100 |
| Pyridoxine hydrochloride | 0.50 |
| Nicotinic acid | 0.50 |
| Glycin | 2.00 |
| Sucrose | 30.0 g (gram) |

<u>Availability in Industry</u> ---

The method for culturing plant organs with an equipment according to the present invention can solve the problems inherent in the conventional culturing method wherein aggregates are frequently produced causing decrease in culture efficiency, and enables uniform and efficient culture of plant organs by virtue of sufficient dispersion of said plant organs inside the culture equipment. The equipment of the present invention is also useful for culturing microorganism.

What is claimed is: ---

1.    A method for culturing plant organs characterized in that an aggregate is disassembled during the course of culturing.

2.    A culture tank for plant organs which comprises at least one agitator capable of disassembling an aggregate of plant organs.

3.    A culture tank for plant organs characterized in that agitation impellers and baffle terminals are installed alternately.

4.    The culture tank as set forth in claim 2 or 3 wherein the clearance between an agitation impeller and the adjacent baffle terminal coincides with the size of plant organs.

Fig. 1

| 1. | Agitator | 2. | Agitation terminal |
|---|---|---|---|
| 3. | Baffle member | 4. | Baffle member terminal |
| 5. | Culture tank | 6. | Air sparger |
| 7. | Exhaust pipe | 8. | Transplantation port |

- 17 -

# INTERNATIONAL SEARCH REPORT

International Application No    PCT/JP89/00306

## I. CLASSIFICATION OF SUBJECT MATTER (if several classification symbols apply, indicate all) [6]

According to International Patent Classification (IPC) or to both National Classification and IPC

$Int.Cl^4$    A01G1/00, C12M3/00

## II. FIELDS SEARCHED

### Minimum Documentation Searched [7]

| Classification System | Classification Symbols |
|---|---|
| IPC | A01G1/00, C12M1/00-1/08,3/00-3/04 C12N5/00-5/02 |

### Documentation Searched other than Minimum Documentation to the Extent that such Documents are Included in the Fields Searched [8]

| | |
|---|---|
| Jitsuyo Shinan Koho | 1926 – 1989 |
| Kokai Jitsuyo Shinan Koho | 1971 – 1989 |

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | JP, A, 57-189689 (Institut biochimii i fiziologii mikroorganizmov Akademii Nauk SSSR) 22 November 1982 (22. 11. 82) &SE, A, 8102585&US, A, 4379846 &CH, A, 651586 | 1 – 4 |
| A | JP, A, 55-15734 (Kyowa Hakko Kogyo Co., Ltd.) 4 February 1980 (04. 02. 80) (Family: none) | 1 |
| A | JP, A, 61-40788 (Albright & Wilson Limited) 27 February 1986 (27. 02. 86) &GB, A, 2162537&EP, A1, 171970 &AU, A, 8545709 | 1 |

[*] Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| June 14, 1989 (14. 06. 89) | July 3, 1989 (03. 07. 89) |
| International Searching Authority | Signature of Authorized Officer |
| Japanese Patent Office | |

Form PCT/ISA/210 (second sheet) (January 1985)